Europäisches Patentamt

European Patent Office

Office européen des brevets

(19)

(11) Veröffentlichungsnummer: **0 236 821**
**B1**

(12) **EUROPÄISCHE PATENTSCHRIFT**

(45) Veröffentlichungstag der Patentschrift:
30.05.90

(51) Int. Cl.⁵: **A61F 2/08**, D03D 1/00

(21) Anmeldenummer: **87102443.6**

(22) Anmeldetag: **20.02.87**

(54) **Künstliches Kreuzband für ein Kniegelenk.**

(30) Priorität: **07.03.86  CH 954/86**

(43) Veröffentlichungstag der Anmeldung:
**16.09.87 Patentblatt 87/38**

(45) Bekanntmachung des Hinweises auf die Patenterteilung:
**30.05.90 Patentblatt 90/22**

(84) Benannte Vertragsstaaten:
**AT DE FR GB IT**

(56) Entgegenhaltungen:
**EP-A- 0 145 492**
**US-A- 4 255 820**

(73) Patentinhaber: **GEBRÜDER SULZER AKTIENGESELLSCHAFT, Zürcherstrasse 9, CH-8401 Winterthur(CH)**

(72) Erfinder: **Stühmer, Karl-Gerhart Dr.-med., St. Elisabethen-Krankenhaus, D-7980 Ravensburg(DE)**
Erfinder: **Frey, Otto, Wallrütistrasse 56, CH-8400 Winterthur(CH)**
Erfinder: **Koch, Rudolf, Oberdorfstrasse 229, CH-8267 Berlingen(CH)**

(74) Vertreter: **Sparing Röhl Henseler Patentanwälte European Patent Attorneys, Rethelstrasse 123, D-4000 Düsseldorf 1(DE)**

## Beschreibung

Die Erfindung betrifft ein künstliches Kreuzband für ein Kniegelenk, das sich aus einem Stamm in mindestens zwei Einzelstränge verzweigt, die das vordere und das hintere Kreuzband bilden.

Ein künstliches Kreuzband der genannten Art ist bekannt aus der FR-A-2 2l3 76l (73.02 342); dieses Kreuzband besteht in seinem Stamm aus einer Anzahl - vorzugsweise aus vier - in Längsrichtung verlaufender,monofiler Fäden aus Polyamid, von denen ab einem Verzweigungspunkt zwei Fäden das vordere und zwei Fäden das hintere Kreuzband bilden. Diese Längsfäden sind von einer Gewebehülle aus bioinertem Kunststoff umhüllt und mit dieser Hülle durch körperverträglichen Klebstoff bzw. durch Nähte verbunden. Um die notwendige Zugfestigkeit zu erreichen, müssen die Fäden eine gewisse Mindestdicke haben. Dadurch werden die elastische Längsdehnbarkeit des Bandes und seine Flexibilität beeinträchtigt. Weiterhin sind diese Längsfäden infolge eines dauernden Reibens aneinander einem relativ hohen Verschleiss unterworfen.

Unverzweigte, ähnliche künstliche Bänder, bei denen eine Gewebehülle einen in Längsrichtung verlaufenden Stamm oder "Längsfaden" umschliessen, sind aus der EP-A-0 l26 520 und aus der US-PS 4 255 820 bekannt.

Aufgabe der Erfindung ist es, ein künstliches Kreuzband zu schaffen, das sich aus einem Stamm heraus in mindestens zwei Äste verzweigt und dessen Längsdehnbarkeit und Flexibilität gegenüber dem dafür bei der eingangs erwähnten Konstruktion erreichten Werten erheblich verbessert sind. Mit der Erfindung wird diese Aufgabe dadurch gelöst, dass der Stamm und die Aeste aus einer Vielzahl textiler Schlauchgebilde bestehen, die schichtweise übereinander gezogen sind; unter "textilen Schlauchgebilden" werden dabei aus multifilen Fäden oder Monofilamenten, beispielsweise Drähten, hergestellte "Hohlzylinder" verstanden, wobei alle für die Verarbeitung von Fäden bekannten Verfahren, wie Flechten, Weben, Stricken, Wirken usw. benutzt werden können, die die geforderten Mindestwerte für die elastische Längsdehnbarkeit und die Flexibilität des Bandes zulassen. Bevorzugt werden die neuen Bänder durch Flechten auf einer Flechtmaschine hergestellt.

Die kernlos aus einer Vielzahl von konzentrischen "Lagen" oder Ringen aufgebauten textilen Schlauchgebilde bewirken eine Vervielfachung der Flexibität und der elastischen Längsdehnbarkeit.

Vor allem für ein geflochtenes verzweigtes Band ergibt sich eine vorteilhafte Ausführungsform, wenn alternierend ein Ring des Schlauchstammes sich in einem und der nächste sich in dem anderen Einzelstrang fortsetzt; es ist jedoch auch möglich, dass die Ringe der inneren Schläuche des Stammes den einen und die äusseren Schlauchringe den anderen Ast bilden. Die geforderte Längsdehnbarkeit und Flexibilität werden beim Aufbau des Bandes aus geflochtenen Schläuchen durch die Einstellung des Flechtwinkels gegen die Bandachse erreicht. Dabei ist die Flexibilität bei einem geflochtenen Band umso grösser je flacher der Flechtwinkel gegen die Bandachse geneigt ist.

Besonders bei gewebten, gewirkten oder gestrickten Bändern besteht weiterhin die Möglichkeit, dass sich jeder einzelne Schlauch in die Einzelstränge verzweigt.

Die einzelnen Schläuche können aus multifilen oder monofilen Fäden hergestellt sein, wobei der Begriff "monofiler Faden" auch metallische Drähte einschliesst. Weiterhin können als Fäden Naturfasern, z.B. Seide, oder Kunstfasern, beispielsweise Polyester, Polyäthylen oder auch bekannte resorbierbare Materialien, verwendet werden. Zur Herstellung eines Bandes werden eine Vielzahl von konzentrischen Schläuchen, beispielsweise 20 - 30, übereinander gezogen oder geschichtet. Dabei haben sich für geflochtene Schläuche gezwirnte Fäden aus einer Vielzahl, z.B. 60, Monofilamenten bewährt, die beispielsweise aus Polyester bestehen und einen Durchmesser von 0,0l bis 0,03 mm haben. Mit Vorteil können äussere Schläuche - um beispielsweise zur Erhöhung der mechanischen Festigkeit und/oder zur Erzeugung einer relativ dichten und glatten Oberfläche möglichst dichte Geflechte zu erhalter-aus einer vergrösserten, beispielsweise verdoppelten, Anzahl Fäden und/oder aus einzelnen Fäden mit einer erhöhten, beispielsweise ebenfalls einer verdoppelten, Anzahl an Monofilamenten gefertigt sein.

Im folgenden wird die Erfindung anhand eines Ausführungsbeispiels im Zusammenhang mit der Zeichnung näher erläutert.

Fig. l stellt das neue Band schematisch in etwa natürlicher Grösse dar;

Fig. 2 ist - gegenüber Fig. l vergrössert - der Schnitt II-II von Fig. l;

Fig. 3 schliesslich ist schematisch eine Ansicht auf ein Kniegelenk von hinten, in das ein erfindungsgemässes Kreuzband implantiert ist.

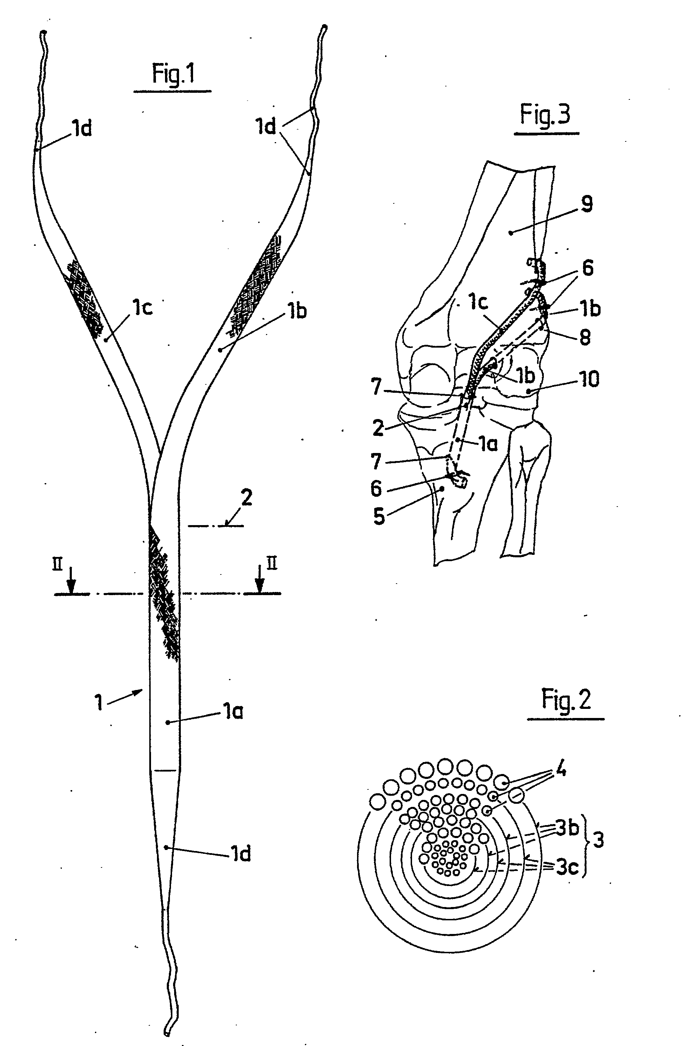

Das als Kreuzbandersatz dienende Band l (Fig. l) hat einen Stamm la, der sich auf einer gewissen "Höhe" 2 in zwei Äste lb und lc verzweigt. An den freien Enden laufen sowohl der Stamm la als auch die Aeste lb und lc in dünne Einfädelabschnitte ld aus, die unter Umständen durch eine Einlage oder durch Tränken in einer härtenden Masse versteift sind.

Wie Fig. 2 zeigt, ist das künstliche Band l kernlos aus einer Vielzahl konzentrischer Schläuche 3 aufgebaut, wobei die einzelnen Fäden 4 der Schläuche 3 in dem gezeigten Beispiel aus einer Vielzahl von Monofilamenten zu einem multifilen Faden verzwirnt sind, was nicht ausdrücklich gezeigt ist.

Das als Beispiel gezeigte Band l ist auf einer Flechtmaschine hergestellt. Die Verzweigung auf der Höhe 2 wird dabei beispielsweise so realisiert, dass der Stamm la alternierend sich bei einem Schlauch 3b (Fig. 2) im Ast lb und beim darauf folgenden Schlauchring 3c im Ast lc fortsetzt. Es ist jedoch auch möglich, den einen Ast aus den inneren und den anderen Ast aus den äusseren Schläuchen 3 des Stammes la zu bilden. Darüberhinaus kann beispielsweise bei gewebten oder gestrickten Schläuchen ein Verzweigen jedes Schlauches in zwei Äste erfolgen.

Um eine relativ dichte und glatte Oberfläche des Bandes I zu erhalten, sind die Fäden 4 äusserer Schläuche dicker, d.h. sie bestehen beispielsweise aus der doppelten Anzahl an Monofilamenten, wie die Fäden der inneren Schläuche.

Mit dem neuen Band ergibt sich eine sehr einfache Implantationstechnik:

Der Stamm la wird mit Hilfe einer Spange oder Agraffe 6, die in den Tibiaknochen 5 eingeschlagen wird, an der Vorderseite des Tibiaknochens 5 (Fig. 3) fixiert, wobei das Band I wenige Zentimeter ausserhalb der Agraffe mit einem scharfen Schneidwerkzeug, beispielsweise einem Skalpell, abgeschnitten und in eine zuvor gesetzte Sackbohrung in dem Tibiaknochen 5 versenkt wird. Vor der Fixierung des Stammes la auf dem Tibiaknochen 5 werden beiden Aeste lb und lc gemeinsam durch eine Bohrung 7 im Tibiaknochen 5 hindurchgeführt; aufgrund des Zuges und infolge der elastischen Längsdehnbarkeit wird dabei das Band I in seinem Durchmesser verkleinert und kann so ohne Schwierigkeiten durch die Bohrung 7 gezogen werden. Beim Nachlassen der Zugspannung quillt es dann auf und hat dadurch in der Bohrung 7 innigen Kontakt mit dem Knochen, wodurch ein An- und Einwachsen von Gewebe gefördert wird.

Die Länge des Stammes la relativ zur Länge der Bohrung 7 im Tibiaknochen 5 wird dabei so gewählt, dass die Verzweigung 2 sich innerhalb der Bohrung 7 befindet.

Nach dem Austritt der Tibiabohrung 7 verläuft der eine Ast lc zwischen den Kondylen (= over the top) und wird lateral aussen am Femur 9 in der gleichen Weise wie der Stamm la in dem Tibiaknochen 5 festgeklemmt; er bildet den Ersatz für das hintere Kreuzband. Der andere Ast lb wird durch eine weitere Bohrung 8, die den Femur oberhalb der lateralen Kondylen l0 durchdringt, ebenfalls auf die laterale Aussenseite des Femurs 9 geleitet und dort auf die beschriebene Art mit einer Agraffe 6 befestigt.

Um die Zugbelastungen auf die Verankerungsstellen zu verringern, wird besonders das "hintere" Kreuzband eine relativ lange Strecke auf dem Femurknochen aufliegend geführt, ehe es verankert wird. Die "lange" Auflage auf dem Knochen 9 ergibt eine erhöhte Reibung, durch die ein Teil der Zugbelastung "verbraucht" und von der Verankerung mit Hilfe der Agraffe 6 ferngehalten wird. Beim Stamm la und beim Ast lc sind die Verankerungsstellen durch den erwähnten Kontakt zwischen dem Band I und dem Knochen 5 bzw. 9 in den Bohrungen 7 bzw. 8 vor Ueberbeanspruchungen geschützt.

## Patentansprüche

l. Künstliches Kreuzband für ein Kniegelenk, das sich aus einem Stamm (1a) in mindestens zwei Einzelstränge (1b, 1c) verzweigt, die das vordere und das hintere Kreuzband bilden, dadurch gekennzeichnet, dass der Stamm (1a) und die Einzelstränge (1b, 1c) aus einer Vielzahl textiler Schlauchgebilde (3) bestehen, die schichtweise konzentrisch übereinander gezogen sind.

2. Kreuzband nach Anspruch 1, dadurch gekennzeichnet, dass sich jeder Schlauch (3) in die Einzelstränge verzweigt.

3. Kreuzband nach Anspruch 1, dadurch gekennzeichnet, dass von jeweils zwei benachbarten Schläuchen (3) des Schlauchstammes (1a) der eine Schlauch (3b) sich in dem einen Einzelstrang (1b) und der andere Schlauch (3c) sich in dem anderen Einzelstrang (1c) fortsetzt.

4. Kreuzband nach Anspruch 1, dadurch gekennzeichnet, dass die inneren Schläuche (3) des Stammes (1a) den einen (1b) und die äusseren Schläuche den anderen Einzelstrang (1c) bilden.

## Claims

1. An artifical cruciate ligament for a knee joint, the ligament branching from a stem (1a) into at least two separate strands (1b, 1c) forming the anterior and posterior cruciate ligament, characterised in that the stem (1a) and the individual strands (1b, 1c) consist of a number of flexible tubular textile structures (3) drawn concentrically one over another in layers.

2. A cruciate ligament according to claim 1, characterised in that each tubular member (3) branches into the individual strands.

3. A cruciate ligament according to claim 1, characterised in that of any two adjacent flexible tubular members (3) of the stem (1a) one flexible tubular member (3b) continues in one individual strand (1b) and the other flexible tubular member (3c) continues in the other individual strand (1c).

4. A cruciate ligament according to claim 1, characterised in that the inner flexible tubular members (3) of the stem (1a) form one individual strand (1b) and the outer flexible tubular members form the other individual strand (1c).

## Revendications

1. Ligament croisé artificiel pour une articulation du genou, qui se ramifie à partir d'un tronc (1a) en au moins deux branches individuelles (1b, 1c) qui constituent le ligament croisé antérieur et le ligament croisé postérieur, caractérisé en ce que le tronc (1a) et les branches individuelles (1b, 1c) sont constitués de plusieurs corps textiles tubulaires (3), qui sont étirés en couches les uns au-dessus des autres.

2. Ligament croisé selon la revendication 1, caractérisé en ce que chaque tube (3) se ramifie dans les branches individuelles.

3. Ligament croisé selon la revendication 1, caractérisé en ce que l'un (3b) de chaque fois deux tubes voisins (3) du tronc tubulaire (1a) se prolonge dans l'une des branches individuelles (1b) et l'autre tube (3c) se prolonge dans l'autre branche individuelle (1c).

4. Ligament croisé selon la revendication 1, caractérisé en ce que les tubes (3) internes du tronc (1a) constituent l'une des branches individuelles (1b) et les tubes externes, l'autre branche individuelle (1c).

Fig.1

Fig.3

Fig.2